# EUROPEAN PATENT APPLICATION

(11) **EP 3 470 116 A1**
(43) Date of publication of application: **17.04.2019**
(21) Application number: 18200534.8
(22) Date of filing: 15.10.2018
(51) Int. Cl.: A61Q 19/00, A61K 8/67, A61K 8/64

(54) **COSMETIC COMPOSITION FOR SKIN AND USE OF A COSMETIC COMPOSITION**

(30) Priority: 14.10.2017 PL 42316117
(71) Applicant: Eveline Cosmetics S.A. Sp. k., 05-506 Lesznowola (PL)
(72) Inventor: KASPRZYCKI, Piotr Hubert, 02-797 Warszawa (PL)
(74) Representative: Marcinska-Porzuc, Aleksandra

(57) **Abstract**

The invention relates to a cosmetic composition for skin, that contains, as active substances, a) d-panthenol from 0.01 to 5% by weight, niacinamide from 0.01 - 5% by weight, calcium d-panthotenate from 0.01 to 5% by weight, hydrolyzed keratin from 0,1 to 5% by weight.

A use of the cosmetic composition for skin as defined above for making a cosmetic composition in a form of a cream/conditioner for hands and nails without age limitation.

A use of the cosmetic composition for skin as defined above for making a cosmetic composition in a form of an emulsion for every skin type, in particular for overdried skin and damaged nails without age limitation.

## Description

This invention relates to a cosmetic composition for hand skin and nails care and use of a cosmetic composition.

Skin constitutes the first protective layer against harmful external factors, it is a waterproof, washable, and elastic coating, and, moreover, it is capable of self-repairing and renewal. The protective function is important especially important for hand skin that is the most important center of the sense of touch. Due to a huge number of nerve endings, the skin receives a great number of various stimuli. Thousands of neural receptors are located on the finger pads, by which we can feel the touch, pressure, heat, pain, etc.

The skin on the back of hand is subjected to squeezing - it can be easily gripped and pulled away. It is necessary to be able to move and bend the fingers. The back of hand is covered by thin, soft hair having a protective function.

The skin covering the palm, so called palm skin, is thick, hairless and rich with tactile receptors and sweat glands that facilitate lowering the body temperature and removing toxins. Due to the sweat glands, that wet the skin, we have better ability of touching and gripping various objects.

The palm skin is significantly different from the skin covering other body parts. The main difference is the minimal thickness of the subcutaneous tissue

This tissue is an energy reservoir for the organism, it consists of fat cells. Thus, it has a supporting function and protects againts any injuries. Because of the lack of the subcutaneous tissue, the palm is especially delicate and sensitive. Additionally, because the layer of the subcutaneous tissue of the hand skin is minimal, its elasticity is also decreased.

Another difference in the structures of the body and the hand skin lies in that the body skin contains both sebaceous glands, that excrete sebum, and sweat glands, that facilitate to remove toxins from the body and maintain the appropriate temperature. On the contrary, the palm skin contains a lot of sweat glands (responsible for the dehydration), but it has no sebaceous glands. This significantly reduces the excretion of sebum and leads to overdrying as well as increased dehydration of the epidermis.

The external portion of the palm skin is directly exposed to environmental factors. The amount of water in the palm epidermis and the dermis increases along with the depth - the longer the distance from the surface, the higher amount of water in the tissues. Dry skin has a lowered amount of water in the epidermis. Dehydration of the stratum corneum increases its permeability and decreases its elasticity. As a consequence, the quality of the epidermal barrier becomes poorer, and the susceptibility to external factors increases. Therefore, it frequently itches, burns, peels, and makes an impression of being shrunk. Irritations, inflammations, infections, allergies, and itchiness may develop. Additionally, callousing may occur and the process of exfoliating the cells of stratum corneum may be disturbed.

Too much dryness is a very frequent reason of the skin problems. Dry skin may be recognized by it is thin, stretched, rough in touch, and sensitive.

Therefore, the appropriate choice of the palm care is very important, especially for dry, sensitive, or atopic skin. It is recommended to wash hands with gentle hand soaps, preferably having a bacteriostatic effect. Additionally, each time after washing the hands, a specially chosen cream should be used having an appropriate composition of emollients, that will wet and elasticise the epidermis intensively, and also will provide appropriate nutritional substances. Also, it should protect against the external factors and regenerate the epidermis, or mitigate any irritation symptoms.

The structure of a nail is quite complex, since it consists of many layers and different components. They may seem to be not very important components but in every day life they have very important functions. Not only because of aesthetic and functional properties but also because of diagnostics. They constitute, inter alia, protective barriers for nerve endings of fingers and toes, and, moreover, they give very good indication basing on which the general health condition may be determined.

From an anatomical point of view, nails are built from epithelium by transforming live skin cells into dead keratinocites. Nail keratic surface, i.e., keratin, is formed after thickening and precise integrating cell walls.

The most important portion of the whole nail organ is the nail matrix. In this region transparent cells are formed that, by tight binding between them, create the nail plate. Too hard strike may damage the matrix causing a disorder of the whole nail growth. The nail plate is the main and the biggest nail component. It is formed from keratine. Due to different density of blood vessels below the plate, it may have slightly different coloration. It rests upon an epidermal portion that constitutes the bed of the nail. It is an element binding the nail plate with the skin. It is the source from which the nail receives all necessary nutrients.

A small white crescent-shaped region may be seen on the nail plate. It s so called moon of the nail. Its color results from not strict adhesion to the matrix. It is most visible on the thumb. The ending of the whole nail constitutes a free edge. Below it, in turn, the hyponychium is located. The whole surface of the nail is surrounded by nail folds. The protect the whole organ. Skin forming the rear nail fold is not ended at the nail's edge but it surfaces creating a nail fold that protects the nail matrix against various infections.

The palm skin, its hydration level, smoothness, and appearance - are the simplest factors indicating the age of a woman. The palms show the first symptoms of the aging process. And because of the special structure of the palm skin (small number of sebaceous glands, and lowered capability of binding and retaining water in the epidermal layer, shortage of lipids in the cornified layer, and decreasing with time production of the collagen result in wrinkles in the palm skin and the skin itself is loosing elasticity), insufficient and improper care (not applying or applying irregularly anti-wrinkle, filling, firming, colagen production stimulating, or protective creams) and the lack of regularity lead to faster loss of firmness and smoothness or worsening of the overall condition of the skin. Stronger tendency to overdrying appears, and dry skin has a higher aging rate. Our lipidic structures of the skin become damaged due to solar radiation, free radicals, nicotine, alcohol, and many other factors. Lipids in the epidermis create a barrier against an invasion of various harmful factors from outside. Free radicals attack proteins in the cell membranes. A cascade of inflammatory processes is started and a cell can not function properly anymore. Collagen and elasticin become damaged. Peptide bond breaks and the protein structure becomes damaged. As a consequence, skin aging occurs significantly faster relative to what is determined by the genetic factors.

Women obsessively care for the face skin, neck skin, and for the body, but they forget that the palms and fingernails are the most visible portions of the body, and that they should pay much more attention thereto. In the summertime we remember, most frequently, about applying an appropriate layer of a cream with a protective filter onto arms and the face only. Also in the winter, we neglect to protect the hands and the nails. Also, we forget about using cosmetics not only for the palm skin but also for the nails and the skin around them. Well-nourished skin around the nails give a healthy appearance to the fingernails.

Apart from the above mentioned behaviors, the palm skin and the nails are also affected by more negative external factors than a face is.

Atmospheric conditions, high or low temperatures, and particularly rapid changes thereof, are the factors that lower the protective barrier capability of the epidermis. When going outdoors in freezing weather from a heated room without mittens, the skin has no chance to adapt to the outdoor conditions. In strong wind or a continuous exposure to harmful solar radiation it s much easier to protect other body parts (protecting them below clothes and using protective filters).

Also at home or at work more harmful chemical factors affect the palm and nail skin than the face skin.

These factors include, first of all, contact of the hands with all sort of detergents, but also a normal soap or wetting the hands. We not always use special protective gloves for household works. Frequent contact with detergents, washing hands with a soap, and additionally with a dish detergent, using electrical dryers for hands instead of paper towels, or too intensive rubbing the skin with a towel, but also staying in an air-conditioned room, all of these not only makes it possible to remove microorganisms and pollutants from the skin surface, but also, unfortunately, removes the natural protective layer of the skin. The hydro-lipidic coating becomes destroyed and the continuity of the epidermis is broken - the skin may break at the finger pads or in the interdigital spaces. One can observe symptoms typical for dry skin - redness, roughness, and unpleasant impression of being shrunk. Overdried skin easily becomes irritated, it is, frequently, supersensitive to cosmetics or even water. Exfoliation and burdensome itchiness may occur.

Also, everyday nail care based on using a color nail polish or applying a nail polish cured by UV lamp (hybrid manicure) does not improve the condition of the nails, and rather it has an adverse effect on the nail plate and the skin. Frequent work with a computer keyboard (hitting against the nail plate) contributes to weakening, damaging, and, as a result, splitting the nails.

A solution to the above mentioned problems is based on regular usage of cosmetics (cosmetic products wear out faster from this body portion, therefore, they should be applied repeatedly) but their composition should be appropriate, i.e., rich with w emolients, special care oils, wetting and regenerative ingredients (i.e., vitamins) affecting both the hand skin as well as the nails. The care should be based on using demulcent agents, greasing the skin and creating an occlusive fatty film stopping dehydration of the epidermis, and having an effect of a soothing pack on the skin, or so frequently recommended for everyday use cream of a thick or even fatty consistency.

Also, one can take special treatments, for example paraffin treatment or mesotherapy, offered by cosmetic studios. Instead of such therapies, one recommends ready-to-use hand packs or, also, a thick layer of a night hand cream applied under cotton gloves.

Therefore, a need exists for intensive hand skin care such that it maintains its firmness and young appearance as long as possible.

To counteract the skin aging, the above mentioned processes should be affected by using products with active substances that will inhibit the process of skin aging by protection and regeneration of the skin.

To maintain the skin in a good condition, the skin needs regular care supplemented with appropriately selected treatments and use of biologically active ingredients that reach deeper layers of the epidermis, having a regenerative, nutritional, and protective effect. By this, the skin is wetted, elastic, and smoothed.

The skin care should be carried out in a multiway manner and should focus on the protective effect - both the restoration of our lipidic layer (hydro-lipidic coating), decrease of the TEWL (Trans Epidermal Water Loss) effect and stimulation of the ceramides production, as well as strengthening the protective barrier, thereby giving better protection against external factors; and the regenerative effect, i.e., repair of possible damages by improving the hydration, elasticity, smoothness and brightness of the skin.

The aim of this invention is to provide cosmetic products containing a composition of active substances having multidirectional effect, affecting most of the aging symptoms of the skin, related both to natural and externally-originated aging processes. The regeneration and protection should be adapted to the needs of the hand skin and nails regardless of the age. The invented cosmetic composition acts as a cream for the skin and a conditioner for the nails.

The subject matter of the invention is a cosmetic composition for skin, that contains, as active substances, a) d-panthenol from 0.01 to 5% by weight, b) niacinamide from 0.01 to 5% by weight, c) calcium d-panthotenate from 0.01 to 5% by weight, and d) hydrolyzed keratin from 0,1 to 5% by weight.

A composition, wherein the weight ratio of the active ingredients a), b), c), and d) is 1:1:1:0.05, correspondingly.

A composition, that contains, additionally, emolients in an amount of 5-20% by weight, hydrating substances in an amount of 0.01-15% by weight, antiaging and smoothening substances in an amount of 0.01 -10% by weight.

A composition that contains emolients selected from a group of: castor oil, soybean oil, paraffinum liquidum.

A composition, that contains antiaging and hydrating substances selected from: hyaluronic acid, betaine, glycerin.

A composition, that contains smoothening substances selected from: hyaluronic acid.

A composition that is in a form of an emulsion for every skin type, in particular for overdried skin and damaged nails without age limitation.

A composition, that has a form of a cream-conditioner for hands for every skin type, in particular for overdried skin and damaged nails without age limitation.

A usage of the cosmetic composition for skin as defined above for making a cosmetic composition in a form of cream/conditioner for hands and nails without age limitation.

A usage of the cosmetic composition for skin as defined above for making a cosmetic composition in a form of an emulsion for every skin type, in particular for overdried skin and damaged nails without age limitation.

D-panthenol - is a natural precursor of vitamin B5 (of pantothenic acid), present in every live tissue. The vitamin B5 itself does not penetrate through the stratum corneum of the epidermis and therefore it is used as a provitamin (D-panthenol) in preparations for external use. Panthenol penetrates the epidermis excellently. It demonstrates a healing effect for various injuries of the skin and mucosae. It soothes irritations and unpleasant symptoms caused by allergenic factors. Vitamin B5 promotes the regeneration of the skin and affects the growth of hair and nails.

Niacinamide (vitamin B3) is a valuable and versatile cosmetic ingredient, the use of which is beneficial for all kinds of skin: dry, oily, acned, capillary, hyperpigmented, mature, with wrinkles, sensitive, irritated, etc. It is an ingredient recommended for every skin type. It stimulates protein synthesis, promotes the blood circulation in the skin and activates the oxygen transport.

Shortage of vitamin B3 in the body manifests as skin diseases, inflammatory conditions, skin redness and roughness, leading, in an advanced stage, to pellagra (abscess and dun spots on the skin). Also, the shortages of vitamin B3 are one of the causes of some tumors, including carcinous symptoms of the skin. In researches, both external as well as internal administration of vitamin B3 led to remission of carcinous symptoms of the skin induced by UV radiation.

In the cosmetology, vitamin B3 is used mainly in a form of niacinamide, since this form is stable, easily water-soluble, well absorbed and tolerated by the skin. It is an ingredient of a high level of safety in use, the activity of which is well examined and proven by many independent studies. It stimulates production of lipidic ingredients of the skin (mainly ceramides) - (Soma Y., Kashima M., Imaizumi A., Takahama H., Kawakami T., Mizoguchi M., Moistirizing effects of topical nicotinamide on atopic dry skin. Int J Derrmatol 2005; 44: 197-202) and of precursors of ingredients responsible for maintaining an appropriate hydration level of the skin (keratines, involucrin, filaggrin), whereby it significantly improves the lipidic barrier of the epidermis, the skin becomes more immune to external factors and better hydrated. It increases hydration and elasticity of the skin, decreases the trans epidermal water loss (TEWL) (Gehring W. Nicotinic acid/niacinamide. J Cosmet Dermatol 2004; 3: 88-93). It has an anti-wrinkle effect, shallows tiny surface wrinkles, smoothens and apparently improves the structure and coloration of the skin (Bissett DL., Miyamoto K., Sun P., Li J., Berge CA., Topical niacinamide reducess yellowing, wrinkling, red blotchiness, and hyperpigmented spots in aging facial skin. Int J Cosmet Sci 2004; 26: 231 - 238). It indirectly affects the production of the collagen and stimulates the synthesis of the hyaluronic acid in the skin, whereby it improves the firmness and the tension of the skin. It is an ingredient assisting a therapy against discoloration of the skin by stopping the migration of the pigment from melanocytes, thus preventing forming visible discoloration spots; it assists other clarifying ingredients. It inhibits yellowing and sallow coloration of the skin developing along with the age and caused by glycation processes, i.e., oxidizing the skin protein. Niacinamide has an anti-glycation effect. It stimulates reparation of skin cell damages caused by UV radiation UV, has an antioxidant function, and has an immunostimulating effect. I assists the function of products with UV filters. It has an antioxidizing effect and promotes regeneration of the skin. It improves the immunity of the skin against external factors, for example detergents, by inhibiting inflammatory cytokines, whereby the skin is less sensitive to irritations (Berson DS., Osborn R., Oblong JE., Hakozaki T., Johnson MB., Bissett DL., Niacinamide: A topical vitamin with wide-ranging skin appearance benefits. Cosmeceuticals and Cosmetic Practise 2014; 10: 103 - 112). It is recommended as an ingredient helpful for atopic skin inflammation and psoriasis, it decreases irritations, redness, improves the protective barrier of the skin. It decreases sebum overproduction and reduces the skin pores size. Also, it is suitable for oily skin.

Calcium Pantothenate, a calcium salt of pantothenic acid, is a complex of a substance constituting vitamin B5 and calcium. It maintains an appropriate hydration level, stimulates cellular growth and regeneration of the tissue. Also, it is a source of calcium for the skin. Calcium is one of most important elements in our body - it has extremely important function in the intercellular communication. This ingredient is necessary in the skin. No production of cytokeratins and no synchronization of cell divisions are possible without it. Natural reduction of calcium level in the body due to aging adversely affects the skin metabolism and regeneration and, as a consequence, its flabbiness and wrinkling.

Calcium pantothenate is a water-soluble substance. It is recommended as an ingredient conditioning the skin, hair, and nails. It accelerates regeneration of the skin, has anti-inflammatory effect, promotes wound healing, improves hydration, etc. It strengthens nails and accelerates their growth. Also, it manifests antistatic and smoothening effects. It gives no unpleasant impression of being sticky that is typical for panthenol. It hydrates excellently, counteracts keratinization of the epidermis, soothes irritations, etc. And, additionally, it contains calcium that, as was proved by Polish researchers from Industrial Chemistry Research Institute, when administered onto the skin, it has an effect on the skin soothing and stimulating growth of cells and regeneration of connective tissue, and counteracts inflammatory states. It is a strong humidifier, it easily penetrates the barrier of the epidermis and enters deeply into the skin. When used regularly, calcium pantothenate hydrates it and creates a protective film on the surface of the epidermis, the skin becomes more elastic, smooth, soft, and hydrated, whereby tiny wrinkles become less visible. Calcium pantothenate is an excellent product for hair and nails overdried with wind, sun, frost, or by treatment procedures - it increases hydration, this causing that adnexa are smooth, soft and have optically increased volume.

When used regularly, calcium pantothenate protects nails against dehydration, gives a gloss, reduces splitting. Additionally, calcium pantothenate demonstrates high affinity to keratin of the skin, hair, and nails, thus it is an excellent remedy for overdried and weak nails.

It is proved that calcium pantothenate promotes hydration of the skin and exhibits wound healing properties (American Journal of Clinical Dermatology, 2002, volume 3, number 6, pp. 427-433). Both d-panthenol as well as calcium pantothenate are administered externally as protective substances in radiotherapy.

Keratin hydrolyzate is a filmforming, hydrophilic, water-soluble substance. It is responsible for maintaining water in the epidermis, whereby it humidifies it, and also conditions it, i.e., softens and smoothens. Keratin has large molecule size and does not penetrate deeply into the skin, staying on the surface where it undergoes a hydrolysis process, i.e., decomposition. Due to this, we obtain smaller protein molecules of a better assimilability by the skin and hair. It exhibits excellent affinity to the skin surface, hair, and nails. Keratin is recommended in care of hair and nails that need regeneration, that are damaged by various treatments, that are brittle, overdried, thin, as well as of healthy hair and nails for protection against external factors and damages. Also, hydrolyzed keratin is used for nails care since it regenerates damaged nail plate, strengthens and thickens it, prevents breaking and splitting nails, hydrates and heals breaking epidermis around the nails, and also improves the condition of yellowed and discolored nails.

Moreover, it has contained emolients (castor oil, soybean oil) - active ingredients improving hydration and oiling of the skin, decreasing the TEWL (trans epidermal water loss) - preventing evaporation from the skin.

Castor oil is pressed from ricinus communis seeds. The active ingredients are undecylenic acid, ricinoleic acid. It contains high levels of unsaturated fatty acids, vitamin E, proteins, and minerals. Traditionally, castor oil has been used for centuries. The first mentions come from ancient Egypt, as a remedy for skin diseases, hair growth, etc. It has anti-inflammatory, antiviral, antibacterial, and fungicidal effects.

Due to unique fatty acids, 90% of which constitutes the ricinoleic acid, when applied onto the skin, it penetrates deeply into the skin, hydrates the skin, and softens it. It stimulates production of the collagen and elastin, improves the hair condition and restores damaged and brittle nails, accelerates their growth, etc. The nails become stronger, stop splitting, and grow faster. It is a rich vitamin E source, that strengthens brittle hair and nails. It is recommended especially for persons who have nails damaged after cosmetic procedures - for example hybrid manicure.

It is a vegetable oil and the plant itself is distributed in Africa and India. Its medical and therapeutic properties make it popular in various fields. Castor oil may be used in many ways for skin care, hair care, and health care.

Castor oil is a blessing for skin problems such as: sunburn, acne, dry skin, and linear atrophy. Also, it is helpful for chronic itch, that may be caused by overdrying the skin after using detergents. It enters deeply and stimulates production of the collagen and elastin. This, in turn, promotes softening and hydrating the skin. Moreover, it inhibits occurring wrinkles and mimic lines, smoothening, softening, and rejuvenating the skin.

Soybean oil is produced from soybean seeds and it is appreciated as a source of proteins and oil. The soybean oil contains ca. 50% of linoleic acid, one of the main EFA acids (essential fatty acid), vitamin E, and lecithin.

The oil content in soybean seeds, in comparison to other oil seeds, is low and reaches 18-24%. Soybean oil contains monounsaturated acids (23-32%), however most of the fatty acids, 50-64%, are polyunsaturated acids, i.e., omega-3 and omega-6 acids, including 2-12% of alpha-linolenic acid.

Soybean oil contains numerous phytosterols, particularly sitosterol that helps to lower the cholesterol level by 10%.

Those are the ingredients that make the cold-pressed soybean oil (unrefined) to have its extraordinary properties.

Soybean oil has yellow color, neutral smell and taste. It contains fatty acids. Contained vitamin E guarantees young and healthy appearance of the skin, and lecithin assists the lipidic protective barrier. Thereby, the skin is appropriately hydrated and oiled. Soybean oil protects the skin against dehydration, whereby hydration level of the skin is appropriate.

Excessive dehydration through the epidermis leads to overdrying the skin and deteriorates its condition - wrinkles and aging symptoms appear faster.

Therefore, complementarily a proved anti-wrinkle agent has been used onto the hand skin and nails - sodium hyaluronate (a sodium salt of hyaluronic acid).

Sodium hyaluronate is an ingredient constituting a structural element of cellular matrix, connecting elastin fibers with collagenic fibers of the dermis.

Along with collagen, they constitute the most important components of the skin. One of few materials used in medicine and cosmetics that is identical to a compound present naturally in the body. In the skin, it is present in so called proteoglycan, where, along with biogenic proteins, it creates its supporting structure. Its content in this system reaches up to 50%. It is a physiological substance, the use of which does not provoke allergies nor irritations. Studies confirm that administering preparations containing sodium hyaluronate, that itself demonstrates a strong hygroscopicity, beneficially affects hydration level and improves the condition of dry skin. It may be used as a hydrating agent both in the summer time (both before and after tanning), as well as in the winter, because it does no expand blood vessels (telangiectasias). By binding water within the epidermis, it makes it smoother, and protects it against drying. Also, this ingredient is used as a protective substance against harmful external factors. It has an ability of transforming toxic substances, e.g., heavy metals or industrial pollutants, into water-soluble complexes, whereby allergy and irritation risk is suppressed. Also, it exhibits a healing and soothing effect. It may be used as a substance participating in creation of biological barriers suppressing penetration of chemical substances and bacteria (it creates a subtle invisible for an eye protective film/coating on the skin).

The cosmetic composition contains, except for the main complex of active ingredients, properly selected emolients, regenerative ingredients, anti-wrinkle substances, smoothening substances, and nail plate strengthening substances improving the skin hydration level, also glycerin and betaine.

The hydrating effect of glycerin is connected with its very strong hygroscopic properties. Glycerin protects the skin in a natural manner by penetrating into intercellular spaces, where it binds water necessary for keeping the appropriate hydration level of the skin. It has excellent soothing properties, it efficiently hydrates overdried skin prone to chapping. It smoothens, improves the elasticity, and regulates the proper regeneration of the epidermis. The effect of glycerin is long-standing. It penetrates into deeper parts of stratum corneum of the epidermis and stays there regulating the humidity for about 24 hours. The presence of glycerin assists dermal lipids.

Betaine is of natural origin from sugar beets. It is a glycin derivative having a hydrating effect and an effect of suppressing irritations of surface layers of the epidermis. Additionally, it protects the skin against water evaporation, it strengthens the protective barrier, whereby skin is more firm.

The cosmetic composition of the cream/conditioner for hands and nails not only contains a unique composition of active substances but also it is based on a specific composition of base substances. The uniqueness of this composition results from using an emulgator (Lauryl Glucoside, Glycerin, Polyglyceryl-2 Dipolyhydroxystearate) recommended, from technological point of view, as a material for spray-type articles (of a low viscosity and liquid consistency) while the created cosmetic composition is a cream/conditioner of a quite high viscosity. The raw material is of organic plant origin having properties that do not irritate the skin. Dermatological studies have been performed proving a very good dermatological profile of the employed emulgator. The raw material in the created cosmetic composition gives very good impressions on the skin - mellowness, softness - and also it leaves a protective coating as well as it decreases the sensitivity of the skin to external factors. Also, due to the material, the skin around the nails is well-cared-for, and the splitting of nail plates is suppressed.

Additionally, the cosmetic composition is, preferably, prepared in a form of an oil emulsion in water, the water content being not less than 50%. Products recommended for the hand skin are emulsions, so called fatty creams - such as water in oil (due to large content of oil phase and emolients) whereby a protective coating is created on the skin - regenerative intercellular cement, suppressing excessive evaporation of water from the epidermis and protecting against external factors. These effects have been obtained due to specific connections of the base substances (for example an organic plant emulgator) and the active ingredients contained in the fabricated oil-in-water type cosmetic composition (so called hydrating) in a form of cream/conditioner for hands and nails. Due to such form, one has no feeling of viscosity and stickiness on the skin, as is the case with fatty creams. The invented cosmetic composition in a form of cream/conditioner, although it is absorbed quickly, suppresses the symptoms typical for dry skin.

The composition according to the invention may contain typical auxiliary substances, media, thickeners, preservatives, colorants, and smell compositions.

One of base ingredients of the cosmetic composition is paraffinum liquidum. Paraffin is a product of oil distillation. It contains substances having oiling properties, but has no nutritional ingredients. Although it does no hydrate itself, it does not enter deeply into the skin, and after being applied, it creates z protective layer that captures the moisture within the skin. One recommends a paraffin treatment for hands to regenerate the hand skin and nails properly.

When repeating such treatments regularly, the nails regenerate, become harder, stop splitting, and even may grow faster.

Thus, it may be said that the use of the ingredients of the cosmetic composition along with a) panthenol b), niacinamide c) calcium d-panthotenate, and d) hydrolyzed keratin gives an effect as a paraffin treatment for hands.

A paraffin treatment comprises steps of skin peeling, rubbing a treatment preparation into the skin, and dipping hands, several times, in a specially prepared and heated paraffin (50-56 C), until obtaining a thick coating. It is necessary to put on gloves keeping the heat. During this heating paraffin bath, the paraffin transmits the heat to the skin and holds it thereon for longer time, by which pores are opened, blood circulation increases, and caring substances present in cosmetic preparations absorb much faster. The duration of the treatment is about 30 minutes. The last step of the treatment is a delicate massage for hands along with application of a nutritional serum. After the paraffin treatment, the hand skin is apparently more soft, more firm, and more hydrated. The paraffin improves the condition of an overdried rough skin. A contraindication to the treatment is osteoporosis and allergy to paraffin. Frequent treatments should be repeated 1 - 2 times per week. Additionally, the paraffin treatments for hands are recommended mainly in the autumn and winter due to the very warming effect.

The advantage of the cosmetic composition for hand skin and nails care in a form of cream/conditioner for hands and nails is the fact that it may be used all year long for persons at any age and any skin type. It induces no thermal effect on the skin. Also, there is no need of visiting a cosmetician and booking time for the treatment. Using the cosmetic composition gives an immediate effect on the skin - the hands appear very naturally and cared for, and the nails are strengthened.

The subject matter of the invention is a cosmetic composition for hand skin and nails care comprising a complex of active substances containing a) panthenol b), niacinamide c) calcium d-panthotenate, and d) hydrolyzed keratin keratin, the composition being capable of replacing paraffin treatments.

In order to increase the effect of the cream/conditioner on the skin and nails, and to improve the skin oiling, and also to promote skin microcirculation, improve the absorption of the active substances contained in the cosmetic composition, the product may be applied and rubbed into the skin. Also, due to the unique cosmetic form, the product is excellent for hand and nails massage.

The cosmetic composition, due to the contents of base and active substances, is recommended for care of dry skin, dehydrated skin, irritated skin, after intensive sun exposure, for weak and splitting nails, etc.

The results of the studies of using the cosmetic composition according to the invention proved that the unique combination of the active ingredients having both protective and regenerative effects significantly contributes to restoration of the epidermis, preserving a young appearance of the hand skin, and strengthening the nails. Additionally, persons taking part in a panel study stated that administering the cosmetic composition gives effects comparable with a professional cosmetic treatment.

The amount of such ingredients within the composition according to the invention will be within limits typically used and recommended by manufacturers.

The invention is illustrated by the following embodiments that are not intended to be consider as a limitation of the invention.

### Example 1

A composition contains: a) d-panthenol in an amount from 0.01 to 5% by weight, b) niacinamide 0.01 - 5% by weight, c) calcium d-panthotenate 0.01 to 5% by weight, and d) hydrolyzed keratin 0,1 to 5% by weight.

Preferably, the active ingredients are present in a weight ratio of a) : b) : c) : d) equal to 1:1:1:0.05.

### Example 2

The subject matter of the invention is a cosmetic composition for hand skin and nails care containing a complex of active substances composed of a) panthenol b), niacinamide c) calcium d-panthotenate, and d) hydrolyzed keratin.

| Ingredient | % | A | B | C |
|---|---|---|---|---|
| Emolients (plant, mineral, or silicone oils), waxes | 5-20 | 5 | 7 | 15 |
| D-panthenol | 0.01-5 | 0.01 | 3 | 5 |
| Niacinamide | 0.01-5 | 0.01 | 2 | 4 |
| Calcium d-pantothenate | 0.01-5 | 0.01 | 3 | 5 |
| Hydrolyzed keratin | 0.1-5 | 0.1 | 0.5 | 3 |
| Hydrating agents | 0.01-15 | 0.5 | 8 | 10 |
| Preservatives | 1-3 | 1 | 2 | 3 |
| Anti-wrinkle smoothening agents | 0.01-10 | 0.01 | 5 | 10 |
| Auxiliary substances, water | Up to 100 | | | |

The preservative substances contained in the cosmetic composition belong to two groups: a group of parabens, a representative of which may be, for example, methylparaben, and a group of hydantoins, such as DMDM hydantoin. Other preservatives may be used such as phenoxyethanol or hydroxyacetophenone.

A thickener belongs to polyacrylates, and, specifically, it is sodium polyacrylate. Other thickeners may be used, for example carbomers or polyacrylamides.

A smell composition contains allergens, for example, Thousand wishes from Charabot S.A.

The emolients are chosen from: castor oil, soybean oil, paraffinum liquidum.

Antiaging and hydrating substances were chosen from: hyaluronic acid, betaine, glycerin.

A smoothening substance is, e.g., hyaluronic acid.

### Application studies:

Studies have been performed according to Regulation (EC) No 1223/2009 of the European Parliament and of the Council of 30 November 2009 on cosmetic products. The studies were based on the Cosmetics Europe - The Personal Care Association Guidelines: product test guidelines for the Assessment of Human Skin Compatibility 1997, guidelines for the evaluation of the Efficacy of Cosmetic Products 2008.

All the studies of the cosmetic compositions have been conducted, under supervision of dermatologists, on human testees - volunteers, in domestic conditions (home panel).

The aim of the research was to determine the efficacy of the cosmetic products. Therefore, a comparative test has been performed, placebo vs the cosmetic composition, as well as an efficacy test for a single product. In every study 60 persons were involved, at the age of 21 to 59, who met the requirements regarding the research and gave aware assent to participate in the research.

The measurements were performed in an air-conditioned room at a temperature of 22°C and air relative humidity of 40 - 60%.

### Bioinstrumental efficacy assessment of the cosmetic composition:

Comparative test placebo vs cosmetic composition:
- A measurement of hydration, the sebum level of the skin surface and TEWL (using instruments from Courage&Khazaka - Multiskincenter 750). Two 1 cm x 1 cm fields were defined on the hand skin for every volunteer. The measurements were done first before application of the product, then after 48 hours from the application, and after 10 days of application.
- A measurement of smoothening (using an instrument Miravex - Antera). The measurements were done first before application of the product, and after 10 days of application.

In addition to the bioinstrumental tests, the volunteers filled a questionnaire related to application properties of the product, and were assessing the efficacy of the product.

All the products were safe in use, the product was well tolerated by the skin and no allergic reactions were observed. None of the persons participating in the study, even having sensitive, prone to irritations, skin, claimed significant subjective complaints, i.e., itching, burning, contracting, etc., that could evidence an adverse influence of the tested product on the skin. Additionally, the products were leaving a protective film on the skin surface that was not giving an impression of sticky skin, this confirming a pleasant impression of using the products. The products were exhibiting very beneficial, multidirectional effects improving the appearance and condition of the skin and nails as soon as after 10 days of using. Therefore, the products may be recommended for everyday care for overdried hand skin suffering from the lack of elasticity and damaged nails, for persons at any age. The tested cosmetic compositions guarantee conspicuous effects - smoothening, hydration of the skin, and long-lasting, up to 48 hours, protection against adverse influence of external factors, i.e., chlorine in water, detergents, microinjuries. This effect will be yet more significant, more noticeable, and beneficial for persons with damaged skin, because the volunteers taking part in the study only occasionally had overdried skin and damaged nails.

The first effects of the cosmetic compositions may be seen after 48 hours from the application (improvement of hydration, sebum, smoothening, and decrease of TEWL), whereas after 10 days of using, the effects are still more evident.

### Comparative test placebo vs cosmetic composition:

The product, after 48 hours from the application, exhibited an effect of hydrating the skin increasing its hydration in the examined group by 14% on average for the cosmetic composition and by 5% for placebo. After 10 days of using, an effect of hydrating the skin in the examined group was increased up to 28% for the cosmetic composition and 11% for placebo.

Due to deep and noticeable hydration, observed by the volunteers during the period of application of the tested cosmetic compositions, the hand skin becomes smoothened and softened.

The study assessing the sebum measurement of the skin demonstrated that the cosmetic composition guarantees a feeling of comfort and protection of the skin. The product, after 48 hours from the application, exhibited a protective effect increasing sebum content of the skin in the examined group by 27% on average for the cosmetic composition and by 4% for placebo. After 10 days of using, sebum level of the skin increased in the examined group up to 48% for the cosmetic composition and 18% for placebo.

A measurement of the transepidermal water loss (TEWL), not only makes it possible to determine, early, the efficacy of the skin water barrier, but also even its smallest damages or even an improvement due to regeneration and sealing the epidermis. One observed a reduction of TEWL by 11% on average after 48 hours from the application in the examined group for the cosmetic composition and 3% for placebo. Whereas after 10 days of using, the decrease of the TEWL value in the examined group for the cosmetic composition was up to 13% and 9% for placebo. Persons participating in the test confirmed that the cosmetic composition helps to restore physiological functions of injured skin and nails, as well as their young and healthy appearance.

Persons participating in the test confirmed that the cosmetic composition, when compared to placebo, has an anti-wrinkle and skin smoothening effect - an improvement of the skin state was observed after 48 hours of using by 9% on average in the examined group for the cosmetic composition and by 3% for placebo. Whereas, after 10 days of using, the skin smoothening effect in the examined group was up to 11% on average for the cosmetic composition and 6% for placebo. It was determined that due to the cosmetic composition, the hand and nail skin was properly cared for, whereby it became more firm, gentle and nice in touch.

In terms of the effectiveness and application properties for nails, the persons participating in the test confirmed that the cosmetic composition, when compared to placebo (Fig. 1), reduces hand dryness, strengthens the nail plate, and improves the condition of skin and nails.

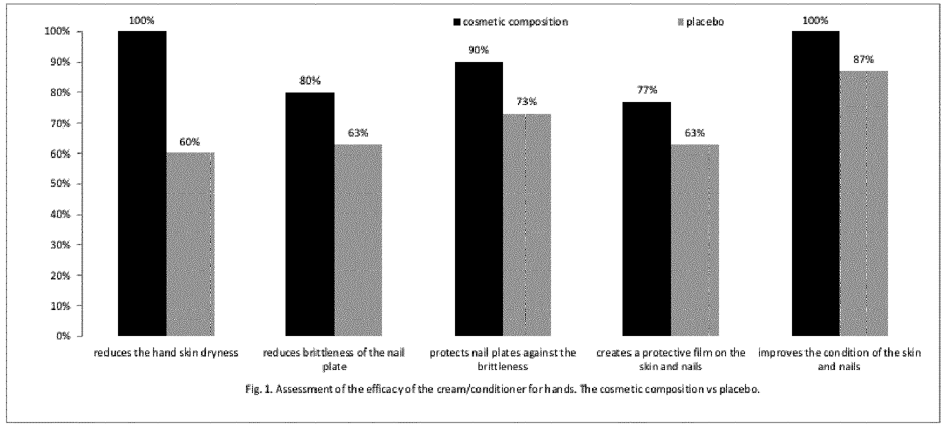

In terms of the effectiveness and application properties for hand skin, an improvement of hydration, nutrition, and regeneration was confirmed by 100% of the volunteers for the cosmetic composition, whereas, for placebo, by 77%, 87%, and 90%, correspondingly (Fig. 2).

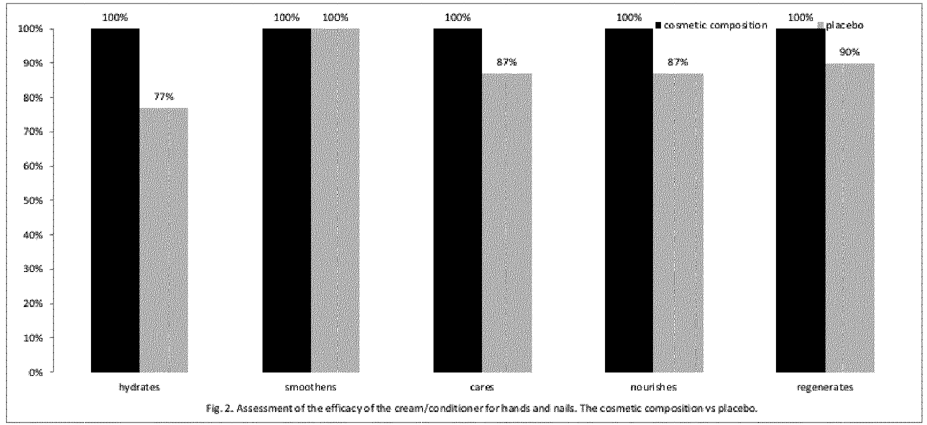

The volunteers, in the questionnaire, stated that the cosmetic composition, due to active ingredients having hydrating properties, efficiently reduces problems related to improper functioning of the skin, resulting from the water shortage. An improvement of the skin hydration improves the firmness, elasticity, and smoothness of the skin. The skin is properly tensioned, strengthened, it does no get sagged whereby its appearance become younger. An improvement of hydration and smoothening contributes to remission and slowing the aging process of the skin. Additionally, persons taking part in the study affirmed that administering the cosmetic composition gives effects comparable with a professional cosmetic treatment.

### Example 3

A method of manufacturing a cosmetic emulsion/cream/composition. First, oil phase ingredients (emolients, emulsifier, castor oil, etc.) are heated up to a temperature of 75-85°C. Then, water phase ingredients, along with preservatives, are heated up to temperature 75-85°C. The water phase comprises colorants, glycerin, betaine, preservatives, sequestrants, d,-panthenol, etc. The phases mix together. Then, the mixture undergoes homogenization, where a thickener is added (time ca. 3-20 minutes). Everything is cooled down to a temperature of 35°C. Then, active ingredients are added (d-panthenol from 0.01 to 5% by weight, niacinamide from 0.01 - 5% by weight, calcium d-panthotenate from 0.01 to 5% by weight, hydrolyzed keratin from 0,1 to 5% by weight), and fragrance composition.

## Claims

1. A cosmetic composition for skin **characterized in that** it contains, as active substances, a) d-panthenol from 0.01 to 5% by weight, niacinamide from 0.01 - 5% by weight, calcium d-panthotenate from 0.01 to 5% by weight, hydrolyzed keratin from 0,1 to 5% by weight.

2. A composition according to claim 1, **characterized in that** the weight ratio of the active ingredients a), b), c), d) is 1:1:1:0.05, correspondingly.

3. A composition according to claim 1 or 2, **characterized in that** it contains, additionally, emolients in an amount of 5-20% by weight, hydrating substances in an amount of 0.01-15% by weight, antiaging substances and smoothening substances in an amount of 0.01 -10% by weight.

4. A composition according to claim 3, **characterized in that** it contains emolients selected from a group of: castor oil, soybean oil, paraffinum liquidum.

5. A composition according to claim 3, **characterized in that** it contains antiaging and hydrating substances selected from: hyaluronic acid, betaine, glycerin.

6. A composition according to claim 3, **characterized in that** the smoothening substance is hyaluronic acid.

7. A composition according to claim 1 -6 **characterized in that** it has a form of an emulsion for every skin type, in particular for overdried skin and damaged nails without age limitation.

8. A composition according to claim 1 - 6 **characterized in that** it has a form of a cream-conditioner for hands for every skin type, in particular for overdried skin and damaged nails without age limitation.

9. A use of the cosmetic composition for skin defined as in claims 1-8 for making a cosmetic composition in a form of a cream/conditioner for hands and nails without age limitation.

10. A use of the cosmetic composition for skin defined as in claims 1-8 for making a cosmetic composition in a form of an emulsion for every skin type, in particular for overdried skin and damaged nails without age limitation.
